(19) **Europäisches Patentamt / European Patent Office / Office européen des brevets**

(11) **EP 3 227 345 B1**

(12) # EUROPÄISCHE PATENTSCHRIFT

(45) Veröffentlichungstag und Bekanntmachung des Hinweises auf die Patenterteilung:
**07.01.2026 Patentblatt 2026/02**

(21) Anmeldenummer: **15801738.4**

(22) Anmeldetag: **24.11.2015**

(51) Internationale Patentklassifikation (IPC):
**C08F 6/22** (2006.01)  **A61L 15/60** (2006.01)
**C08F 2/18** (2006.01)  **A61L 15/22** (2006.01)
**C08J 3/24** (2006.01)  **C08F 220/06** (2006.01)
**B01J 20/26** (2006.01)  **B01J 20/30** (2006.01)

(52) Gemeinsame Patentklassifikation (CPC):
(C-Sets verfügbar)
**B01J 20/265; A61L 15/60; B01J 20/3085; C08F 2/18; C08F 6/22; C08J 3/245;** C08J 2333/02
(Forts.)

(86) Internationale Anmeldenummer:
**PCT/EP2015/077431**

(87) Internationale Veröffentlichungsnummer:
**WO 2016/087262 (09.06.2016 Gazette 2016/23)**

(54) **VERFAHREN ZUR HERSTELLUNG WASSERABSORBIERENDER POLYMERPARTIKEL DURCH SUSPENSIONSPOLYMERISATION**

METHOD FOR PRODUCING WATER-ABSORBING POLYMER PARTICLES BY SUSPENSION POLYMERIZATION

PROCÉDÉ DE FABRICATION DE PARTICULES POLYMÈRES HYDRO-ABSORBANTES PAR POLYMÉRISATION EN SUSPENSION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.12.2014 EP 14196175**

(43) Veröffentlichungstag der Anmeldung:
**11.10.2017 Patentblatt 2017/41**

(73) Patentinhaber: **BASF SE**
**67056 Ludwigshafen am Rhein (DE)**

(72) Erfinder:
• **MARK, Tina**
**67454 Hassloch (DE)**
• **DANIEL, Thomas**
**67165 Waldsee (DE)**
• **LUTZ, Erich**
**67122 Altrip (DE)**
• **MOLTER, Stefan**
**67133 Maxdorf (DE)**
• **KOWALSKI, Anna**
**67227 Frankenthal (DE)**

(74) Vertreter: **BASF IP Association**
**BASF SE**
**GBI - Z078**
**67056 Ludwigshafen (DE)**

(56) Entgegenhaltungen:
| | |
|---|---|
| EP-A2- 1 433 526 | WO-A1-2006/014031 |
| WO-A1-2008/026783 | WO-A1-2015/110321 |
| WO-A2-2015/062883 | US-A1- 2011 059 329 |
| US-A1- 2011 238 026 | |

Bemerkungen:
Die Akte enthält technische Angaben, die nach dem Eingang der Anmeldung eingereicht wurden und die nicht in dieser Patentschrift enthalten sind.

(52) Gemeinsame Patentklassifikation (CPC): (Forts.)

C-Sets
C08F 220/06, C08F 222/103

**Beschreibung**

Die vorliegende Erfindung betrifft ein Verfahren zur Herstellung wasserabsorbierender Polymerpartikel durch Suspensionspolymerisation und thermischer Oberflächennachvernetzung, wobei das durch Suspensionspolymerisation erhaltende agglomerierte Grundpolymer eine Zentrifugenretentionskapazität von mindestens 37 g/g aufweist und die thermische Oberflächennachvernetzung bei 100 bis 190° C durchgeführt wird.

Die Herstellung wasserabsorbierender Polymerpartikel wird in der Monographie "Modern Superabsorbent Polymer Technology", F.L. Buchholz und A.T. Graham, Wiley-VCH, 1998, Seiten 69 bis 117, beschrieben. Die wasserabsorbierenden Polymerpartikel werden üblicherweise durch Lösungspolymerisation oder Suspensionspolymerisation hergestellt.

Wasserabsorbierende Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Eigenschaften der wasserabsorbierenden Polymere können über den Vernetzungsgrad eingestellt werden. Mit steigendem Vernetzungsgrad steigt die Gelfestigkeit und sinkt die Absorptionskapazität.

Zur Verbesserung der Anwendungseigenschaften, wie beispielsweise Permeabilität im gequollenen Gelbett in der Windel und Absorption unter Druck, werden wasserabsorbierende Polymerpartikel im allgemeinen oberflächennachvernetzt. Dadurch steigt nur der Vernetzungsgrad der Partikeloberfläche, wodurch die Absorption unter Druck und die Zentrifugenretentionskapazität zumindest teilweise entkoppelt werden können.

JP S63-218702 beschreibt ein kontinuierliches Verfahren zur Herstellung wasserabsorbierender Polymerpartikel durch Suspensionspolymerisation.

WO 2006/014031 A1 beschreibt ein Verfahren zur Herstellung wasserabsorbierender Polymerpartikel durch Suspensionspolymerisation. Bei den deutlich höheren Temperaturen bei der thermischen Nachvernetzung wird der Anteil an hydrophobem Lösungsmittel ausgetrieben. Die erhaltenen Superabsorber weisen eine Zentrifugenretentionskapazität (CRC) von deutlich weniger als 37 g/g auf.

WO 2008/068208 A1 betrifft ebenfalls ein Verfahren zur Herstellung wasserabsorbierender Polymerpartikel mit niedrigem Anteil hydrophober Lösungsmittel durch Suspensionspolymerisation.

US 2011/0238026 A1, US 2011/0059329 A1 und WO 2015/110321 A1 offenbaren jeweils Verfahren zur Sprühpolymerisation. Die Agglomeration in einem hydrohoben Lösungsmittel wird nicht offenbart.

WO 2015/062883 A2 offenbart ein Verfahren zur umgekehrten Suspensionspolymerisation. Es wird nicht in einem hydrophoben Lösungsmittel agglomeriert.

WO 2008/026783 A1 offenbart ein Verfahren zur Herstellung von Superabsorbern mit einem hohen "pressurized void average radius index". Die Superabsorber können auch durch umgekehrte Suspensionspolymerisation hergestellt werden. Die erhaltenen Superabsorber weisen eine Zentrifugenretentionskapazität (CRC) von deutlich weniger als 37 g/g auf.

EP 1 433 526 A2 betrifft Superabsorbermischungen aus einem Superabsorber (R1) und einem Superabsorber (R2). Der Superabsorber (R1) wird durch Lösungspolymerisation hergestellt und der Superabsorber (R2) durch umgekehrte Suspensionspolymerisation. Es wird nicht in einem hydrophoben Lösungsmittel agglomeriert und die Zentrifugenretentionskapazität (CRC) von mindestens 37 g/g oder die Absorption unter einem Druck von 49,2 g/cm$^2$ (AUHL) von mindestens 14 g/g werden nicht erreicht.

Aufgabe der vorliegenden Erfindung war die Bereitstellung eines verbesserten Verfahrens zur Herstellung wasserabsorbierender Polymerpartikel durch Suspensionspolymerisation, wobei die wasserabsorbierenden Polymerpartikel eine hohe Zentrifugenretentionskapazität (CRC), eine hohe Absorption unter einem Druck von 49,2 g/cm$^2$ (AUHL), eine hohe Summe von Zentrifugenretentionskapazität (CRC) und Absorption unter einem Druck von 49,2 g/cm$^2$ (AUHL), sowie wenig Extrahierbare aufweisen sollen.

Gelöst wurde die Aufgabe durch ein Verfahren zur Herstellung wasserabsorbierender Polymerpartikel durch Polymerisation einer Monomerlösung, enthaltend

a) mindestens ein ethylenisch ungesättigtes, säuregruppentragendes Monomer, das zumindest teilweise neutralisiert sein kann,
b) optional einen oder mehrere Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,

wobei die Monomerlösung während der Polymerisation in einem hydrophoben organischem Lösungsmittel suspendiert ist, das hydrophobe organische Lösungsmittel bei 23° C eine Löslichkeit in Wasser von weniger als 5 g/100 g aufweist, während oder nach der Polymerisation im hydrophoben organischen Lösungsmittel agglomeriert wird und thermischer Oberflächennachvernetzung der erhaltenen agglomerierten Polymerpartikel mittels eines organischen Oberflächennachvernetzers, dadurch gekennzeichnet, dass die Menge an Vernetzer b) so gewählt wird, dass die agglomerierten Polymerpartikel vor der Oberflächennachvernetzung eine Zentrifugenretentionskapazität von mindestens 37 g/g aufweisen und die thermische Oberflächennachvernetzung bei 100 bis 190° C durchgeführt wird.

In einer bevorzugten Ausführungsform der vorliegenden Erfindung wird die Menge an Vernetzer b) so gewählt, dass die agglomerierten Polymerpartikel vor der Oberflächennachvernetzung eine Zentrifugenretentionskapazität von mindestens 38 g/g aufweisen und wird die thermische Oberflächennachvernetzung bei 105 bis 180° C durchgeführt.

In einer besonders bevorzugten Ausführungsform der vorliegenden Erfindung wird die Menge an Vernetzer b) so gewählt, dass die agglomerierten Polymerpartikel vor der Oberflächennachvernetzung eine Zentrifugenretentionskapazität von mindestens 39 g/g aufweisen und wird die thermische Oberflächennachvernetzung bei 110 bis 175° C durchgeführt.

In einer ganz besonders bevorzugten Ausführungsform der vorliegenden Erfindung wird die Menge an Vernetzer b) so gewählt, dass die agglomerierten Polymerpartikel vor der Oberflächennachvernetzung eine Zentrifugenretentionskapazität von mindestens 40 g/g aufweisen und wird die thermische Oberflächennachvernetzung bei 120 bis 170° C durchgeführt.

Die Monomeren a) sind vorzugsweise wasserlöslich, d.h. die Löslichkeit in Wasser bei 23° C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Säuregruppen der Monomere a) können teilweise neutralisiert sein. Die Neutralisation wird auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Methylenbisacrylamid, Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind Methylenbisacrylamid und die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Methylenbisacrylamid, Di- und/oder Triacrylate des 3- bis 10-fach ethoxylierten Glyzerins. Ganz besonders bevorzugt sind Methylenbisacrylamid, Di- oder Triacrylate des 1- bis 5-fach ethoxylierten und/oder propoxylierten Glyzerins. Am meisten bevorzugt sind Methylenbisacrylamid und die Triacrylate des 3- bis 5-fach ethoxylierten und/oder propoxylierten Glyzerins, insbesondere Methylenbisacrylamid und das Triacrylat des 3-fach ethoxylierten Glyzerins.

Die Menge an Vernetzer in der Monomerlösung wird so gewählt, dass die wasserabsorbierenden Polymerpartikel nach der Polymerisation und vor der thermischen Oberflächennachvernetzung (Grundpolymer) eine Zentrifugenretentionskapazität (CRC) von mindestens 37 g/g, vorzugsweise mindestens 38 g/g, besonders bevorzugt mindestens 39 g/g, ganz besonders bevorzugt mindestens 40 g/g, aufweisen. Die Zentrifugenretentionskapazität (CRC) sollte 75 g/g nicht übersteigen. Bei einer zu hohen Zentrifugenretentionskapazität (CRC) des Grundpolymers kann bei der folgenden thermischen Oberflächennachvernetzung keine hinreichende Absorption unter einem Druck von 49,2 g/cm$^2$ (AUHL) mehr aufgebaut werden.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren.

Geeignete Redox-Initiatoren sind Kalium- bzw. Natriumperoxodisulfat/Ascorbinsäure, Wasserstoffperoxid/Ascorbinsäure, Kalium- bzw. Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Kalium- bzw. Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich.

Geeignete thermische Initiatoren sind insbesondere Azoinitiatoren, wie 2,2'-Azobis[2-(2-imidazolin-2-yl)propan]dihydrochlorid und 2,2'-Azobis[2-(5-methyl-2-imidazolin-2-yl)propan]dihydrochlorid, 2,2'-Azobis(2-amidinopropan) dihydrochlorid, 4,4'-Azobis(4-cyano-pentansäure), 4,4' und deren Natriumsalze, 2,2'-Azobis[2-methyl-N-(2-hydroxyethyl)propionamid] und 2,2'-Azobis(-imino-1-pyrrolidino-2-ethylpropans)dihydrochlorid.

Geeignete Photoinitiatoren sind beispielsweise 2-Hydroxy-2-methylpropiophenon und 1-[4-(2-Hydroxyethoxy)-phenyl]-2-hydroxy-2-methyl-1-propan-1-on.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacry-lat, Dimethylaminoethylmethacrylat, Dimethylaminoethylacrylat, Dimethylaminopropylacrylat, Diethylaminopropylacry-lat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Optional können der Monomerlösung oder ihren Ausgangsstoffen ein oder mehrere Chelatbildner zur Maskierung von Metallionen, wie beispielsweise Eisen, zwecks Stabilisierung zugesetzt werden. Geeignete Chelatbildner sind beispielsweise Alkalicitrate, Zitronensäure, Alkalitartrate, Pentanatriumtriphosphat, Ethylendiamintetraazetat, Nitrilotriessigsäure, sowie alle unter dem Namen Trilon® bekannten Chelatbildner, wie beispielsweise Trilon® C (Pentanatriumdiethylentriaminpentaazetat), Trilon® D (Trinatrium-(hydroxyethyl)-ethylen-diamintriazetat), sowie Trilon® M (Methylglycindiessigsäure).

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden.

Wird die Polymerisation unter ausreichendem Rückfluss durchgeführt, so kann auf die Inertisierung verzichtet werden. Dabei wird der gelöste Sauerstoff zusammen mit dem verdampfenden Lösungsmittel aus dem Polymerisationsreaktor entfernt.

Zur Polymerisation wird die Monomerlösung in einem hydrophoben Lösungsmittel suspendiert bzw. emulgiert.

Als hydrophobe Lösungsmittel sind alle dem Fachmann zum Einsatz bei der Suspensionspolymerisation bekannten Lösungsmittel einsetzbar. Bevorzugt werden aliphatische Kohlenwasserstoffe, wie n-Hexan, n-Heptan, n-Oktan, n-Nonan, n-Dekan, Cyclohexan oder Mischungen daraus, verwendet. Hydrophobe Lösungsmittel weisen bei 23° C eine Löslichkeit in Wasser zu weniger als 5 g/100 g, vorzugsweise weniger als 1 g/100 g, besonders bevorzugt weniger als 0,5 g/100 g, auf.

Das hydrophobe Lösungsmittel siedet im Bereich von vorzugsweise 50 bis 150° C, besonders bevorzugt 60 bis 120° C, ganz besonders bevorzugt 70 bis 90° C.

Das Verhältnis zwischen hydrophoben Lösungsmittel und Monomerlösung beträgt 0,2 bis 3,0 bevorzugt 0,3 bis 2,7 und ganz bevorzugt von 0,4 bis 2,4.

Zur Dispergierung der wässrigen Monomerlösung im hydrophoben Lösungsmittel bzw. zur Dispergierung der entstehenden wasserabsorbierenden Polymerpartikeln können Dispergierhilfsmittel zugesetzt werden. Es kann sich dabei um anionische, kationische, nichtionische oder amphotere Tenside, oder natürliche, halbsynthetische oder synthetische Polymere handeln.

Anionische Tenside sind beispielsweise Natriumpolyoxyethylendodecylethersulfat und Natriumdodecylethersulfat. Ein kationisches Tensid ist beispielsweise Trimethylstearylammoniumchlorid. Ein amphoteres Tensid ist beispielsweise Carboxymethyldimethylcetylammonium. Nichtionische Tenside sind beispielsweise Saccharosefettsäureester, wie Saccharosemonostearat und Saccharosedilaurat, Sorbitanester, wie Sorbitanmonostearat, Trehalosefettsäureester, wie Trehalosestearinsäureester, Polyoxyalkylen-Verbindungen auf Basis von Sorbitanestern, wie Polyoxyethylensorbitanmonostearat.

Geeignete Polymere sind beispielsweise Zellulosederivate, wie Hydroxyethylzellulose, Methylhydroxyethylzellulose, Methylhydroxypropylcellulose, Methylzellulose und Carboxymethylzellulose, Polyvinylpyrrolidon, Copolymere des Vinylpyrrolidons, Gelatine, Gummiarabicum, Xanthan, Kasein, Polyglycerole, Polyglycerolfettsäureester, Polyethylenglykole, modifiziertes Polyethylenglykol, wie Polyethylenglykolstearat oder Polyethylenglykolstearyletherstearat Polyvinylalkohol, partiell hydrolysierte Polyvinylacetate und modifiziertes Polyethylen, wie ein mit Maleinsäure modifiziertes Polyethylen.

Es können aber auch anorganische Partikel als Dispergierhilfsmittel verwendet werden, sogenannte Pickering-Systeme. Ein solches Pickering-System kann dabei aus den festen Teilchen allein oder zusätzlich aus Hilfsstoffen bestehen, die die Dispergierbarkeit der Partikel in Wasser oder die Benetzbarkeit der Partikel durch das hydrophobe Lösungsmittel verbessern. Die Wirkweise und ihre Verwendung werden in WO 99/24525 A1 und EP 1 321 182 A1 beschrieben

Die anorganischen festen Partikel können Metallsalze sein, wie Salze, Oxide und Hydroxide von Kalzium, Magnesium, Eisen, Zink, Nickel, Titan, Aluminium, Silizium, Barium und Mangan. Zu nennen sind Magnesiumhydroxid, Magnesiumkarbonat, Magnesiumoxid, Kalziumoxalat, Kalziumkarbonat, Bariumkarbonat, Bariumsulfat, Titandioxid, Aluminiumoxid, Aluminiumhydroxid und Zinksulfid. Silikate, Bentonit, Hydroxyapatit und Hydrotalcite seien ebenfalls genannt. Besonders bevorzugt sind auf $SiO_2$-basierende Kieselsäuren, Magnesiumpyrophosphat und Trikalziumphosphat.

Geeignete auf $SiO_2$-basierende Dispergierhilfsmittel sind hochdisperse Kieselsäuren. Sie können als feine, feste Teilchen in Wasser dispergiert werden. Es ist aber auch möglich, sogenannte kolloidale Dispersionen von Kieselsäure in Wasser zu verwenden. Solch kolloidale Dispersionen sind alkalische, wässrige Mischungen von Kieselsäure. Im alkalischen pH-Bereich sind die Partikel gequollen und in Wasser stabil. Bevorzugte kolloidale Dispersionen von Kieselsäure haben bei pH 9,3 eine spezifische Oberfläche im Bereich von 20 bis 90 m$^2$/g.

Weiterhin können beliebige Mischungen der Dispergierhilfsmittel eingesetzt werden.

Das Dispergierhilfsmittel wird üblicherweise im hydrophoben Lösungsmittel gelöst oder dispergiert. Das Dispergiermittel wird in Mengen zwischen 0.01 und 10 Gew.-%, bevorzugt zwischen 0,2 und 5 Gew.-%, besonders bevorzugt zwischen 0,5 und 2 Gew.-%, bezogen auf die Monomerlösung, eingesetzt. Über Art und Menge des Dispergierhilfsmittels kann der Durchmesser der Monomerlösungstropfen eingestellt werden.

Der Durchmesser der Monomerlösungstropfen kann über die eingetragene Rührenergie und durch geeignete Dispergierhilfsmittel eingestellt werden.

Die Durchführung der Agglomeration ist dem Fachmann bekannt und unterliegt keinen Beschränkungen. Die Polymerisation und die Agglomeration können gleichzeitig (einstufige Dosierung) oder nacheinander (zweistufige Dosierung) durchgeführt werden.

Bei der einstufigen Dosierung wird die Monomerlösung in das hydrophobe Lösungsmittel dosiert und die Monomerlösungstropfen agglomerieren während der Polymerisation.

Bei der zweistufigen Dosierung wird zuerst eine erste Monomerlösung in das hydrophobe Lösungsmittel dosiert und die Monomerlösungstropfen polymerisiert. Zu den so erhaltenen dispergierten Polymerpartikeln wird anschließend eine zweite Monomerlösung dosiert und erneut polymerisiert. Die Polymerpartikel agglomerieren erst bei der zweiten Polymerisation. Die erste und die zweite Monomerlösung können von ihrer Zusammensetzung identisch oder verschieden sein.

Mit jeder weiteren Monomerzugabe zu bereits gebildeten Agglomeraten, unabhängig davon ob diese durch einstufige oder zweistufige Dosierung hergestellt wurden, können die Agglomerate weiter zu größeren Agglomeraten agglomeriert werden.

Zwischen den Monomoerdosierungen können Kühlschritte liegen. Ein Teil des Dispergierhilfsmittels kann dabei ausfallen.

Ob die Monomerlösungstropfen bereits während der Polymerisation agglomerieren oder nicht, kann über Art und Menge des Dispergierhilfsmittels eingestellt werden. Bei einer ausreichenden Menge an Dispergierhilfsmittel wird die Agglomeration während der Polymerisation der Monomerlösungstropfen verhindert. Die dazu notwendige Menge hängt von der Art des Dispergierhilfsmittels ab.

Die zweistufige Dosierung, d.h. die Agglomeration nach der Polymerisation der Monomerlösungstropfen, ist bevorzugt.

Vorteilhaft werden für die Polymerisation mehrere Rührreaktoren hintereinander geschaltet. Durch die Nachreaktion in weiteren Rührreaktoren kann der Monomerumsatz erhöht und die Rückvermischung vermindert werden. Hierbei ist es weiterhin vorteilhaft, wenn der erste Rührreaktor nicht zu groß ist. Mit steigender Größe des Rührreaktors verbreitert sich zwangsläufig die Größenverteilung der dispergierten Monomerlösungstropfen. Ein kleinerer erster Reaktor ermöglicht daher die Herstellung wasserabsorbierender Polymerpartikel mit besonders enger Partikelgrößenverteilung.

Die Reaktion wird vorzugsweise unter vermindertem Druck durchgeführt, beispielsweise bei einem Druck von 800 mbar. Über den Druck kann der Siedepunkt der Reaktionsmischung auf die gewünschte Reaktionstemperatur eingestellt werden.

In einer bevorzugten Ausführungsform der vorliegenden Erfindung wird die Polymerisation in Gegenwart eines üblicherweise wasserlöslichen Kettenübertragungsreagenz durchgeführt.

Kettenübertragungsreagenzien greifen in die Polymerisationskinetik ein und regeln das Molgewicht. Geeignete Kettenübertragungsreagenzien sind Thiole, Thiolsäuren, sekundäre Alkohole, Phosphorverbindungen, Milchsäure, Aminocarbonsäuren, usw.

Das Kettenübertragungsreagenz wird in einer Menge von vorzugsweise 0,00001 bis 0,1 mol/mol, besonders bevorzugt von 0,00015 bis 0,08 mol/mol, ganz besonders bevorzugt 0,0002 bis 0,06 mol/mol, eingesetzt, jeweils bezogen auf Monomer a).

Die erhaltenen wasserabsorbierenden Polymerpartikel werden thermisch oberflächennachvernetzt. Die thermische Oberflächennachvernetzung kann in der Polymerdispersion oder mit den aus der Polymerdispersion abgetrennten und getrockneten wasserabsorbierenden Polymerpartikeln durchgeführt werden.

Zugabe der Monomerlösung kann auch oberhalb des Siedepunktes von Wasser bzw. des Lösungmittels oder des Lösungsmittel-Wasser-Azeotrops liegen, so dass während der Monomerzugabe kontinuierlich Lösungsmittel oder ein Lösungsmittel-Wasser-Azeotrop abdestilliert wird.

In einer bevorzugten Ausführungsform der vorliegenden Erfindung werden die wasserabsorbierenden Polymerpartikel in der Polymerdispersion azeotrop entwässert, aus der Polymerdispersion filtriert, die filtrierten wasserabsorbierenden Polymerpartikel zur Entfernung des anhaftenden restlichen hydrophoben Lösungsmittels getrocknet und thermisch oberflächennachvernetzt.

Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des Weiteren sind in DE 40 20 780 C1 Alkylenkarbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Weiterhin können auch Oberflächennachvernetzer eingesetzt werden, die zusätzliche polymerisierbare ethylenisch ungesättigte Gruppen enthalten, wie in DE 37 13 601 A1 beschrieben.

Weiterhin können beliebige Mischungen der geeigneten Oberflächennachvernetzer eingesetzt werden.

Bevorzugte Oberflächennachvernetzer sind Alkylenkarbonate, 2-Oxazolidinone, Bis- und Poly-2-oxazolidinone, 2-Oxotetrahydro-1,3-oxazine, N-Acyl-2-Oxazolidinone, zyklische Harnstoffe, bizyklische Amidoacetale, Oxetane, Bisoxetane und Morpholin-2,3-dione.

Besonders bevorzugte Oberflächennachvernetzer sind Ethylenkarbonat (1,3-Dioxolan-2-on), Trimethylenkarbonat (1,3-Dioxan-2-on), 3-Methyl-3-oxethanmethanol, 2-Hydroxyethyl-2-oxazolidinon, 2-Oxazolidinon und Methyl-2-oxazolidinon.

Ganz besonders bevorzugt ist Ethylenkarbonat.

Die Menge an Oberflächennachvernetzer beträgt vorzugsweise 0,1 bis 10 Gew.-%, besonders bevorzugt 0,5 bis 7,5 Gew.-%, ganz besonders bevorzugt 1 bis 5 Gew.-%, jeweils bezogen auf die Polymerpartikel.

Die Oberflächennachvernetzer werden typischerweise als wässrige Lösung eingesetzt. Die Menge des Lösungsmittels beträgt vorzugsweise 0,001 bis 8 Gew.-%, besonders bevorzugt 2 bis 7 Gew.-%, ganz besonders bevorzugt 3 bis 6 Gew.-%, und insbesondere 4 bis 5 Gew.-%, jeweils bezogen auf die Polymerpartikel. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 10:90 bis 60:40 beträgt.

In einer bevorzugten Ausführungsform der vorliegenden Erfindung werden vor, während oder nach der thermischen Oberflächennachvernetzung zusätzlich zu den Oberflächennachvernetzern Kationen, insbesondere polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Hydroxid, Chlorid, Bromid, Sulfat, Hydrogensulfat, Karbonat, Hydrogenkarbonat, Nitrat, Phosphat, Hydrogenphosphat, Dihydrogenphosphat und Carboxylat, wie Acetat, Citrat und Lactat, möglich. Es sind auch Salze mit unterschiedlichen Gegenionen möglich, beispielsweise basische Aluminiumsalze, wie Aluminiummonoacetat oder Aluminiummonolaktat. Aluminiumsulfat, Aluminiummonoacetat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

In einer weiteren bevorzugten Ausführungsform der vorliegenden Erfindung werden vor, während oder nach der thermischen Oberflächennachvernetzung zusätzlich Hydrophilisierungsmittel aufgebracht, beispielsweise Zuckeralkohole, wie Sorbitol, Mannitol und Xylitol, wasserlösliche Polymere oder Copolymere, wie Zellulose, Polyethylenglykole, Polyvinylalkohole, Polyvinylpyrrolidone und Polyacrylamide.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch oberflächennachvernetzt.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die thermische Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die thermische Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und thermisch oberflächennachvernetzt.

Für die thermische Oberflächennachvernetzung kann es vorteilhaft sein, dieses im Unterdruck durchzuführen oder diesen unter Verwendung von Trocknungsgasen, wie beispielsweise getrocknete Luft und Stickstoff durchzuführen, um die möglichst vollständige Entfernung der Lösungsmittel zu gewährleisten.

Anschließend können die oberflächennachvernetzten Polymerpartikel klassiert werden, wobei zu kleine und/oder zu große Polymerpartikel abgetrennt und in das Verfahren rückgeführt werden.

Die Oberflächennachvernetzung kann auch in der Polymerdispersion durchgeführt werden. Dazu wird die Lösung des Oberflächennachvernetzers der Polymerdispersion zugesetzt. Dabei kann es vorteilhaft sein die thermische Oberflächennachvernetzung im Überdruck durchzuführen, beispielsweise bei Verwendung von hydrophoben organischen Lösungsmitteln mit einem Siedepunkt bei 1013 mbar unterhalb der gewünschten Temperatur für die thermische Oberflächennachvernetzung. Nach der thermischen Oberflächennachvernetzung in der Polymerdispersion werden die wasserabsorbierenden Polymerpartikel in der Polymerdispersion azeotrop entwässert, aus der Polymerdispersion abgetrennt, die abgetrennten wasserabsorbierenden Polymerpartikel zur Entfernung des anhaftenden restlichen hydrophoben Lösungsmittels getrocknet.

Die Oberflächennachvernetzungstemperaturen liegen im Bereich 100 bis 190° C, vorzugsweise im Bereich von 105 bis 180° C, besonders bevorzugt im Bereich von 110 bis 175° C, ganz besonders bevorzugt im Bereich von 120 bis 170° C. Die bevorzugte Verweilzeit bei dieser Temperatur beträgt vorzugsweise mindestens 10 Minuten, besonders bevorzugt mindestens 20 Minuten, ganz besonders bevorzugt mindestens 30 Minuten, und üblicherweise höchstens 120 Minuten.

In einer bevorzugten Ausführungsform der vorliegenden Erfindung werden die wasserabsorbierenden Polymerpartikel nach der thermischen Oberflächennachvernetzung im Kontakttrockner gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die wasserabsorbierenden Polymerpartikel auf 20 bis 150° C, vorzugsweise 30 bis 120° C, besonders bevorzugt 40 bis 100° C, ganz besonders bevorzugt 50 bis 80° C, abgekühlt.

Die im Kontakttrockner thermisch oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80° C, besonders bevorzugt bei 35 bis 70° C, ganz besonders bevorzugt bei 40 bis 60° C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20 und Plantacare 818 UP und Tensidmischungen.

Ein weiterer Gegenstand der vorliegenden Erfindung sind die gemäß dem erfindungsgemäßen Verfahren erhältlichen wasserabsorbierenden Polymerpartikel.

Die gemäß dem erfindungsgemäßen Verfahren erhältliche wasserabsorbierenden Polymerpartikel weisen eine Zentrifugenretentionskapazität (CRC) von mindestens 37 g/g, eine Absorption unter einem Druck von 21,0 g/cm$^2$ von mindestens 30 g/g, eine Absorption unter einem Druck von 49,2 g/cm$^2$ (AUHL) von mindestens 14 g/g, eine Summe aus Zentrifugenretentionskapazität und Absorption unter einem Druck von 21,0 g/cm$^2$ (CRC+AUL) von mindestens 69 g/g, eine Summe aus Zentrifugenretentionskapazität und Absorption unter einem Druck von 49,2 g/cm$^2$ (CRC+AUHL) von mindestens 54 g/g und weniger als 20 Gew.-% Extrahierbare aufweisen

Die erfindungsgemäßen wasserabsorbierenden Polymerpartikel weisen eine Zentrifugenretentionskapazität (CRC) von vorzugsweise mindestens 38 g/g, besonders bevorzugt mindestens 40 g/g, ganz besonders bevorzugt mindestens 41 g/g, auf. Die Zentrifugenretentionskapazität (CRC) der wasserabsorbierenden Polymerpartikel beträgt üblicherweise weniger als 75 g/g.

Die erfindungsgemäßen wasserabsorbierenden Polymerpartikel weisen eine Absorption unter einem Druck von 21,0 g/cm$^2$ (AUL) von vorzugsweise mindestens 32 g/g, besonders bevorzugt mindestens 33 g/g, ganz besonders bevorzugt mindestens 34 g/g, auf. Die Absorption unter einem Druck von 21,0 g/cm$^2$ (AUL) der wasserabsorbierenden Polymerpartikel beträgt üblicherweise weniger als 50 g/g.

Die erfindungsgemäßen wasserabsorbierenden Polymerpartikel weisen eine Absorption unter einem Druck von 49,2 g/cm$^2$ (AUHL) von vorzugsweise mindestens 16 g/g, besonders bevorzugt mindestens 18 g/g, ganz besonders bevorzugt mindestens 20 g/g, auf. Die Absorption unter einem Druck von 49,2 g/cm$^2$ (AUHL) der wasserabsorbierenden Polymerpartikel beträgt üblicherweise weniger als 35 g/g.

Die Summe aus Zentrifugenretentionskapazität (CRC) und Absorption unter einem Druck von 21,0 g/cm$^2$ (AUL) der erfindungsgemäßen wasserabsorbierenden Polymerpartikel beträgt vorzugsweise mindestens 71 g/g, besonders bevorzugt mindestens 73 g/g, ganz besonders bevorzugt mindestens 74 g/g.

Die Summe aus Zentrifugenretentionskapazität (CRC) und Absorption unter einem Druck von 49,2 g/cm$^2$ (AUHL) der erfindungsgemäßen wasserabsorbierenden Polymerpartikel beträgt vorzugsweise mindestens 56 g/g, besonders bevorzugt mindestens 58 g/g, ganz besonders bevorzugt mindestens 59 g/g.

Die erfindungsgemäßen wasserabsorbierenden Polymerpartikel enthalten vorzugsweise weniger als 17 Gew.-%, besonders bevorzugt weniger als 15 Gew.-%, ganz besonders bevorzugt weniger als 14 Gew.-%, an Extrahierbaren.

Die erfindungsgemäßen wasserabsorbierenden Polymerpartikel haben einen Anteil an Partikeln mit einer Partikelgröße von 300 bis 600 μm von vorzugsweise mindestens 30 Gew.-%, besonders bevorzugt mindesten 40 Gew.-%, ganz besonders bevorzugt mindestens 50 Gew.-%.

Ein weiterer Gegenstand der vorliegenden Erfindung sind Hygieneartikel, umfassend

(A) eine obere flüssigkeitsdurchlässige Schicht,

(B) eine untere flüssigkeitsundurchlässige Schicht,

(C) eine flüssigkeitsabsorbierende Speicherschicht zwischen der Schicht (A) und der Schicht (B), enthaltend von 0 bis 30 Gew.-% eines Fasermaterial und von 70 bis 100 Gew.-% gemäß dem erfindungsgemäßen Verfahren erhältliche wasserabsorbierende Polymerpartikel,

(D) optional eine Aufnahme- und Verteilschicht zwischen der Schicht (A) und der Schicht (C), enthaltend von 80 bis 100 Gew.-% eines Fasermaterial und von 0 bis 20 Gew.-% gemäß dem erfindungsgemäßen Verfahren erhältliche wasserabsorbierende Polymerpartikel,

(E) optional eine Gewebeschicht direkt über und/oder unter der Schicht (C) und

(F) weitere optionale Komponenten.

Der Anteil an gemäß dem erfindungsgemäßen Verfahren erhältlichen wasserabsorbierende Polymerpartikeln in der flüssigkeitsabsorbierenden Speicherschicht (C) beträgt vorzugsweise mindestens 75 Gew.-%, besonders bevorzugt mindestens 80 Gew.-%, ganz besonders bevorzugt mindestens 90 Gew.-%.

Die mittlere Sphärizität der gemäß dem erfindungsgemäßen Verfahren erhältlichen wasserabsorbierende Polymerpartikeln in der flüssigkeitsabsorbierenden Speicherschicht (C) beträgt vorzugsweise weniger als 0,84, besonders bevorzugt weniger als 0,82, ganz besonders bevorzugt weniger als 0,80.

Wasserabsorbierende Polymerpartikel mit relativ niedriger Sphärizität werden durch Suspensionspolymerisation erhalten, wenn die Polymerpartikel während oder nach der Polymerisation agglomeriert werden. In den erfindungsgemäßen Hygieneartikeln werden agglomerierte wasserabsorbierende Polymerpartikel eingesetzt.

Die wasserabsorbierenden Polymerpartikel werden mittels der nachfolgend beschriebenen Testmethoden geprüft.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymere werden vor der Messung gut durchmischt.

Restmonomer

Der Gehalt an Restmonomer der wasserabsorbierenden Polymerpartikel wird gemäß der von der EDANA empfohlenen Testmethode WSP Nr. 210.2-05 "Residual Monomers" bestimmt.

Feuchtegehalt

Der Feuchtegehalt der wasserabsorbierenden Polymerpartikel wird gemäß der von der EDANA empfohlenen Testmethode Nr. WSP 230.3 (11) "Mass Loss Upon Heating" bestimmt.

Zentrifugenretentionskapazität (Centrifuge Retention Capacity)

Die Zentrifugenretentionskapazität (CRC) wird gemäß der von der EDANA empfohlenen Testmethode Nr. WSP 241.3 (11) "Fluid Retention Capacity in Saline, After Centrifugation" bestimmt.

Absorption unter einem Druck von 0,0 g/cm²

Die Absorption unter einem Druck von 0,0 g/cm² (AUNL) wird analog der von der EDANA empfohlenen Testmethode Nr. WSP 242.3 (11) "Gravimetric Determination of Absorption Under Pressure" bestimmt, wobei statt eines Drucks von 21,0 g/cm² (AUL0.3psi) ein Druck von 0,0 g/cm² (AUL0.0psi) eingestellt wird.

Absorption unter einem Druck von 21,0 g/cm$^2$

Die Absorption unter einem Druck von 21,0 g/cm$^2$ (AUL) der wasserabsorbierenden Polymerpartikel wird gemäß der von der EDANA empfohlenen Testmethode Nr. WSP 242.3 (11) "Gravimetric Determination of Absorption Under Pressure" bestimmt.

Absorption unter einem Druck von 49,2 g/cm$^2$

Die Absorption unter einem Druck von 49,2 g/cm$^2$ (AUHL) wird analog der von der EDANA empfohlenen Testmethode Nr. WSP 242.3 (11) "Gravimetric Determination of Absorption Under Pressure" bestimmt, wobei statt eines Drucks von 21,0 g/cm$^2$ (AUL0.3psi) ein Druck von 49,2 g/cm$^2$ (AUL0.7psi) eingestellt wird.

Schüttgewicht

Das Schüttgewicht wird gemäß der von der EDANA empfohlenen Testmethode Nr. WSP 250.3 (11) "Gravimetric Determination of Density" bestimmt.

Extrahierbare

Der Gehalt an extrahierbaren Bestandteilen der wasserabsorbierenden Polymerpartikel wird gemäß der von der EDANA empfohlenen Testmethode Nr. WSP 270.3 (11) "Extractable" bestimmt. Die Extraktionszeit beträgt 16 Stunden.

Anquellgeschwindigkeit (Free Swell Rate)

Zur Bestimmung der Anquellgeschwindigkeit (FSR) werden 1,00 g (= W1) der wasserabsorbierenden Polymerpartikel in ein 25 ml Becherglas eingewogen und gleichmäßig auf dessen Boden verteilt. Dann werden 20 ml einer 0,9 gew.-%igen Kochsalzlösung mittels eines Dispensers in ein zweites Becherglas dosiert und der Inhalt dieses Glases wird dem ersten zügig hinzugefügt und eine Stoppuhr gestartet. Sobald der letzte Tropfen Salzlösung absorbiert wird, was man am Verschwinden der Reflexion auf der Flüssigkeitsoberfläche erkennt, wird die Stoppuhr angehalten. Die genaue Flüssigkeitsmenge, die aus dem zweiten Becherglas ausgegossen und durch das Polymer im ersten Becherglas absorbiert wurde, wird durch Rückwägung des zweiten Becherglases genau bestimmt (=W2). Die für die Absorption benötigte Zeitspanne, die mit der Stoppuhr gemessen wurde, wird als t bezeichnet. Das Verschwinden des letzten Flüssigkeitstropfens auf der Oberfläche wird als Zeitpunkt t bestimmt.

Daraus errechnet sich die Anquellgeschwindigkeit (FSR) wie folgt:

$$FSR\ [g/g\ s] = W2/(W1 x t)$$

Wenn der Feuchtegehalt der wasserabsorbierenden Polymerpartikel jedoch mehr als 3 Gew.-% beträgt, so ist das Gewicht W1 um diesen Feuchtegehalt zu korrigieren.

Vortex-Test

In ein 100 ml-Becherglas, welches ein Magnetrührstäbchen der Größe 30 mm x 6 mm enthält, werden 50,0 ml $\pm$ 1,0 ml einer 0,9 Gew.-%igen wässrigen Kochsalzlösung gegeben. Mit Hilfe eines Magnetrührers wird die Kochsalzlösung bei 600 Upm gerührt. Es werden dann möglichst schnell 2,000 g $\pm$ 0,010 g wasserabsorbierende Polymerpartikel zugegeben, und die Zeit gemessen, die vergeht, bis die Rührtraube durch die Absorption der Kochsalzlösung durch die wasserabsorbierenden Polymerpartikel verschwindet. Dabei kann der ganze Inhalt des Becherglases sich als einheitliche Gelmasse immer noch drehen, aber die Oberfläche der gelierten Kochsalzlösung darf keine individuellen Turbulenzen mehr zeigen. Die benötigte Zeit wird als Vortex berichtet.

Beispiele:

Herstellung des Grundpolymers:

Beispiel 1

In ein 2 L-Planschliffgefäß, ausgestattet mit Impellerrüher und Rückflusskühler, wurden 340,00 g Heptan und 0,92 g Saccharosestearat (Ryoto® Sugar Ester S-370, Mitsubishi Chemical Europe GmbH, Düsseldorf, Deutschland) vorgelegt und auf 70° C erhitzt bis das Saccharosestearat vollständig gelöst war.

Eine Monomerlösung (erste Dosierung), hergestellt aus 73,40 g (1,019 mol) Acrylsäure, 61,20 g (0,765 mol) 50gew.-%ige wässrige Natriumhydroxidlösung, 109,5 g Wasser und 0,11 g (0,407 mmol) Kaliumperoxodisulfat, wurde daraufhin in ein Zulaufgefäß gefüllt und mit Luft gespült. Unmittelbar vor dem Zutropfen der Monomerlösung, bei einer Rührerdrehzahl von 300 U/min, wurde die Lösung mittels Stickstoffeinleitung inertisiert und eine Ölbadtemperatur von 55° C eingestellt.

Nach Zulaufende wurde eine Stunde bei 70° C weitergerührt, die Reaktionslösung anschließend auf ca. 25° C abgekühlt und eine eisgekühlte Monomerlösung (zweite Dosierung), hergestellt aus 95,90 g (1,331 mol) Acrylsäure, 79,30 g (0,991 mol), 50gew.-%ige wässrige Natriumhydroxidlösung, 143,10 g Wasser und 0,14 g (0,518 mmol) Kaliumperoxodisulfat in ein Zulaufgefäß gefüllt und mit Luft gespült. Unmittelbar vor dem Zutropfen der Monomerlösung, bei einer Rührerdrehzahl von 300 U/min, wurde die Lösung mittels Stickstoffeinleitung inertisiert. Die Monomerlösung wurde innerhalb von 15 Minuten zugetropft.

Nach Zulaufende wurde eine Ölbadtemperatur von 70° C eingestellt. 120 Minuten nach Aufheizbeginn wurde der Rückflusskühler gegen einen Wasserauskreiser ausgetauscht und Wasser ausgekreist.

Die vorliegende Suspension wurde auf 60° C abgekühlt und die erhaltenen Polymerpartikel wurden über einen Büchnertrichter mit Papierfilter abgesaugt. Die weitere Trocknung erfolgte bei 45° C im Umlufttrockenschrank und ggf. im Vakuumtrockenschrank bei 800 mbar bis zu einem Restfeuchtegehalt von kleiner 15 Gew.-%.

Die Eigenschaften der erhaltenen Polymerpartikel sind in Tabelle 2 zusammengefasst.

Beispiele 2 und 3

Die Herstellung des Grundpolymers erfolgte analog zu Beispiel 1 mit den in der Tabelle 1 angegebenen Mengenangaben.

Die Eigenschaften der erhaltenen Polymerpartikel sind in Tabelle 2 zusammengefasst.

Beispiel 4

Die Herstellung des Grundpolymers erfolgte analog zu Beispiel 1 mit den in der Tabelle 1 angegebenen Mengenangaben, wobei die erste Monomerlösung (erste Dosierung) zusätzlich 3,0 g 2-Propanol (Isopropanol) enthielt.

Die Eigenschaften der erhaltenen Polymerpartikel sind in Tabelle 2 zusammengefasst.

Beispiel 5

In ein 2 L-Planschliffgefäß, ausgestattet mit Impellerrüher und Rückflusskühler, wurden 896,00 g Cylohexan, 2,00 g Span® 20 (Sorbitanmonolaurat), 3,20 g Tixoge|® VZ (organophiles Bentonit) und 20,0 g einer 0,015 %igen wässrigen Ascorbinsäurelösung vorgelegt und unter Rühren und Stickstoffeinleitung auf 75° C Innentemperatur erhitzt.

Eine Monomerlösung, hergestellt aus 150,00 g (2,082 mol) Acrylsäure, 125,10 g (1,613 mol) 50gew.-%ige wässrige Natriumhydroxidlösung, 138 g Wasser, 0,0375 g (0,243 mmol) N,N'-Methylenbisacrylamid (MBA) und 0,5 g (1,850 mmol) Kaliumperoxdisulfat, wurde daraufhin in ein Zulaufgefäß gefüllt und mit Luft gespült. Unmittelbar vor dem Zutropfen der Monomerlösung, bei einer Rührerdrehzahl von 300 U/min, wurde die Lösung mittels Stickstoffeinleitung inertisiert. Während der gesamten Zeit der Monomerdosierung wurden die Rückflussbedingungen beibehalten. Die Monomerlösung wurde innerhalb von 60 Minuten zugetropft.

Nach Zulaufende schloss sich eine Nachreaktionszeit von 60 Minuten an. Anschließend wurde der Rückflusskühler gegen einen Wasserauskreiser ausgetauscht und Wasser ausgekreist.

Die vorliegende Suspension wurde auf 60° C abgekühlt und die erhaltenen Polymerpartikel wurden über einen Büchnertrichter mit Papierfilter abgesaugt. Die weitere Trocknung erfolgte bei 45° C im Umlufttrockenschrank und ggf. im Vakuumtrockenschrank bei 800 mbar bis zu einem Restfeuchtegehalt von kleiner 15 Gew.-%.

Die Eigenschaften der erhaltenen Polymerpartikel sind in Tabelle 2 zusammengefasst.

Beispiel 6

In ein 2 L-Planschliffgefäß, ausgestattet mit Impellerrüher und Rückflusskühler, wurden 896,00 g Cylohexan, 2,00 g Span® 20 (Sorbitanmonolaurat), 3,20 g Tixogel® VZ (organophiles Bentonit) und 20,0 g einer 0,015 %igen wässrigen Ascorbinsäurelösung vorgelegt und unter Rühren und Stickstoffeinleitung auf 75° C Innentemperatur erhitzt.

Eine Monomerlösung, hergestellt aus 150,00 g (2,082 mol) Acrylsäure, 118,0 g (1,475 mol) 50gew.-%ige wässrige Natriumhydroxidlösung, 136,8 g Wasser, 0,075 g (0,194 mmol) des Triacrylats des 3-fach ethoxylierten Glyzerins (Gly-(EO-AA)$_3$) und 0,5 g (1,850 mmol) Kaliumperoxdisulfat, wurde daraufhin in ein Zulaufgefäß gefüllt und mit Luft gespült. Unmittelbar vor dem Zutropfen der Monomerlösung, bei einer Rührerdrehzahl von 300 U/min, wurde die Lösung mittels Stickstoffeinleitung inertisiert. Während der gesamten Zeit der Monomerdosierung wurden die Rückflussbedingungen beibehalten. Die Monomerlösung wurde innerhalb von 60 Minuten zugetropft.

Nach Zulaufende schloss sich eine Nachreaktionszeit von 60 Minuten an. Anschließend wurde der Rückflusskühler gegen einen Wasserauskreiser ausgetauscht und Wasser ausgekreist.

Die vorliegende Suspension wurde auf 60° C abgekühlt und die erhaltenen Polymerpartikel wurden über einen Büchnertrichter mit Papierfilter abgesaugt. Die weitere Trocknung erfolgte bei 45° C im Umlufttrockenschrank und ggf. im Vakuumtrockenschrank bei 800 mbar bis zu einem Restfeuchtegehalt von kleiner 15 Gew.-%.

Die Eigenschaften der erhaltenen Polymerpartikel sind in Tabelle 2 zusammengefasst.

Tabelle 1: Einsatzmengen an Vernetzer b)

| Bsp. | Schritt/Zulauf | Vernetzer b) | g | mmol | ppm boaa | mmol% boaa |
|---|---|---|---|---|---|---|
| 1 | 1 | MBA | 0 | 0 | 0 | 0 |
| | 2 | MBA | 0 | 0 | 0 | 0 |
| 2 | 1 | MBA | 0,009 | 0,06 | 125 | 6 |
| | 2 | MBA | 0,012 | 0,08 | 125 | 6 |
| 3 | 1 | MBA | 0,018 | 0,12 | 250 | 12 |
| | 2 | MBA | 0,023 | 0,15 | 250 | 12 |
| 4 | 1 | MBA*) | 0,018 | 0,12 | 250 | 12 |
| | 2 | MBA*) | 0,023 | 0,15 | 250 | 12 |
| 5 | - | MBA**) | 0,0375 | 0,24 | 250 | 12 |
| 6 | - | Gly-(EO-AA)$_3$**) | 0,075 | 0,19 | 500 | 9 |

*) zusätzlich Isopropanol als Kettenübertragungsreagenz
**) einstufige Dosierung
boaa: bezogen auf unneutralisierte Acrylsäure (based on acrylic acid)
MBA: Methylenbisacrylamid
Gly-(EO-AA)$_3$ Triacrylat des 3-fach ethoxylierten Glyzerins

Tabelle 2: Eigenschaften der wasserabsorbierenden Polymerpartikel (Grundpolymer)

| Bsp. | CRC g/g | AUNL g/g | AUL g/g | AUHL g/g | Schüttdichte g/100 ml | Feuchtegehalt % | Extrahierbare % |
|---|---|---|---|---|---|---|---|
| 1 | 55,0 | 44,3 | 7,2 | 6,6 | 89 | 2,2 | 34 |
| 2 | 46,1 | 54,1 | 7,3 | 6,7 | 78 | 4,5 | 21 |
| 3 | 42,8 | 52,0 | 7,4 | 6,5 | 79 | 4,4 | 21 |
| 4 | 48,4 | 54,1 | 7,8 | 7,2 | 83 | 4,0 | 20 |
| 5 | 40,7 | 39,9 | 7,5 | 6,6 | 70 | 3,5 | 26 |
| 6 | 64,0 | 9,7 | 6,9 | 6,1 | 91 | 2,0 | 29 |

Thermische Oberflächennachvernetzung:

Beispiele 1-1 und 1-2

20 g Grundpolymer aus Beispiel 1 wurden in einen Mischer vom Typ Waring®-Blender 32BL80 (8011) eingefüllt. Anschließend wurde der Mischer auf Stufe 1 eingeschaltet. Unmittelbar danach wurden 1,5 g einer wässrige Lösung, bestehend aus 0,5 g Ethylenkarbonat und 1,0 g Wasser, gemäß Tabelle 3, in einer Spritze eingefüllt und innerhalb von 2 Sekunden in den Mischer zudosiert. Nach 3 Sekunden wurde der Mischer ausgeschaltet und die erhaltenen Polymerpartikel wurden gleichmäßig in einer Glasschale mit einem Durchmesser von 20 cm verteilt. Zur thermischen Oberflächennachvernetzung wurde die mit den Polymerpartikeln gefüllte Glasschale in einem Umlufttrockenschrank bei 160° C für 60 bzw. 120 Minuten getempert. Die Polymerpartikel wurde in eine kalte Glasschale umgefüllt. Zum Schluss wurden die gröberen Partikel mit einem Sieb mit einer Maschenweite von 850 $\mu$m entfernt.

Die Eigenschaften der Polymerpartikel sind in Tabelle 4 zusammengefasst.

Beispiele 2-1 und 2-2

Die thermische Oberflächennachvernetzung erfolgte analog zu Beispiel 1-1 und 1-2, aber unter Verwendung des Grundpolymers aus Beispiel 2. Die Temperatur im Umlufttrockenschrank betrug 160° C. Die Temperzeit betrug 60 bzw. 120 Minuten. Die Bedingungen sind in Tabelle 3 zusammengefasst.
Die Eigenschaften der Polymerpartikel sind in Tabelle 4 zusammengefasst.

Beispiele 3-1 und 3-2

Die thermische Oberflächennachvernetzung erfolgte analog zu Beispiel 1-1 und 1-2, aber unter Verwendung des Grundpolymers aus Beispiel 3. Die Temperatur im Umlufttrockenschrank betrug 160° C. Die Temperzeit betrug 60 bzw. 120 Minuten. Die Bedingungen sind in Tabelle 3 zusammengefasst.
Die Eigenschaften der Polymerpartikel sind in Tabelle 4 zusammengefasst.

Beispiele 3-3 und 3-4

Die thermische Oberflächennachvernetzung erfolgte analog zu Beispiel 1-1, aber unter Verwendung des Grundpolymers aus Beispiel 3 und Verwendung von N,N,N',N'-Tetrakis(2-hydroxyethyl)ethylendiamin (Primid® XL 552) als Oberflächennachvernetzer. Die Temperatur im Umlufttrockenschrank betrug 160° C. Die Temperzeit betrug 60 Minuten. Die Bedingungen sind in Tabelle 3 zusammengefasst.
Die Eigenschaften der Polymerpartikel sind in Tabelle 4 zusammengefasst.

Beispiele 3-5 und 3-6

Die thermische Oberflächennachvernetzung erfolgte analog zu Beispiel 1-1, aber unter Verwendung des Grundpolymers aus Beispiel 3. Die Temperatur im Umlufttrockenschrank betrug 90° C bzw. 200° C. Die Temperzeit betrug 60 Minuten. Die Bedingungen sind in Tabelle 3 zusammengefasst.
Die Eigenschaften der Polymerpartikel sind in Tabelle 4 zusammengefasst.

Beispiele 4-1 und 4-2

Die thermische Oberflächennachvernetzung erfolgte analog zu Beispiel 1-1 und 1-2, aber unter Verwendung des Grundpolymers aus Beispiel 4. Die Temperatur im Umlufttrockenschrank betrug 160° C. Die Temperzeit betrug 60 bzw. 120 Minuten. Die Bedingungen sind in Tabelle 3 zusammengefasst.
Die Eigenschaften der Polymerpartikel sind in Tabelle 4 zusammengefasst.

Beispiele 5-1 und 5-2

Die thermische Oberflächennachvernetzung erfolgte analog zu Beispiel 1-1 und 1-2, aber unter Verwendung des Grundpolymers aus Beispiel 5. Die Temperatur im Umlufttrockenschrank betrug 160° C. Die Temperzeit betrug 60 bzw. 120 Minuten. Die Bedingungen sind in Tabelle 3 zusammengefasst.
Die Eigenschaften der Polymerpartikel sind in Tabelle 4 zusammengefasst.

Beispiele 6-1 und 6-2

Die thermische Oberflächennachvernetzung erfolgte analog zu Beispiel 1-1 und 1-2, aber unter Verwendung des Grundpolymers aus Beispiel 6. Die Temperatur im Umlufttrockenschrank betrug 160° C. Die Temperzeit betrug 60 bzw. 120 Minuten. Die Bedingungen sind in Tabelle 3 zusammengefasst.
Die Eigenschaften der Polymerpartikel sind in Tabelle 4 zusammengefasst.

Beispiel 7

Example 1 aus US 8,003,210 wurde nachgearbeitet.
Die Eigenschaften der Polymerpartikel sind in Tabelle 4 zusammengefasst.

Tabelle 3: Thermische Oberflächennachvernetzung im Waring®-Blender - Bedingungen

| Bsp. | Vernetzer b) | Temperatur ° C | Zeit min | Ethylen-karbonat Gew.-% bop | Ethylenkarbonat mmol pro 20 g Polymer | Wasser Gew.-% bop | Primid® XL 552 Gew.-% bop | Primid® XL 552 mmol pro 20 g Polymer |
|---|---|---|---|---|---|---|---|---|
| 1 | 0 ppm MBA | - | - | - | - | - | - | - |
| 1-1 | | 160 | 60 | 2,5 | 5,68 | 5 | - | - |
| 1-2 | | 160 | 120 | 2,5 | 5,68 | 5 | - | - |
| 2 | 125 ppm MBA | - | - | - | - | - | - | - |
| 2-1 | | 160 | 60 | 2,5 | 5,68 | 5 | - | - |
| 2-2 | | 160 | 120 | 2,5 | 5,68 | 5 | - | - |
| 3 | 250 ppm MBA | - | - | - | - | - | - | - |
| 3-1 | | 160 | 60 | 2,5 | 5,68 | 5 | - | - |
| 3-2 | | 160 | 120 | 2,5 | 5,68 | 5 | - | - |
| 3-3 | | 160 | 60 | - | - | 5 | 4,55 | 2,84 |
| 3-4 | | 160 | 60 | - | - | 5 | 9,05 | 5,68 |
| 3-5 | | 90 | 60 | 2,5 | 5,68 | 5 | | |
| 3-6 | | 200 | 60 | 2,5 | 5,68 | 5 | | |
| | | | | | | | | |
| | | | | | | | | |
| | | | | | | | | |
| 4 | 250 ppm MBA*) | - | - | - | - | - | - | - |
| 4-1 | | 160 | 60 | 2,5 | 5,68 | 5 | - | - |
| 4-2 | | 160 | 120 | 2,5 | 5,68 | 5 | - | - |
| 5 | 250 ppm MBA**) | - | - | - | - | - | - | - |
| 5-1 | | 160 | 60 | 2,5 | 5,68 | 5 | - | - |
| 5-2 | | 160 | 120 | 2,5 | 5,68 | 5 | - | - |
| 6 | 500 ppm Gly-(EO-AA)$_3$**) | - | - | | - | - | - | - |
| 6-1 | | 160 | 60 | 2,5 | 5,68 | 5 | - | - |
| 6-2 | | 160 | 120 | 2,5 | 5,68 | 5 | - | - |

bop: bezogen auf Grundpolymer (based on polymer)
*) zusätzlich Isopropanol als Kettenübertragungsreagenz
**) einstufig agglomeriert

Tabelle 4: Thermische Oberflächennachvernetzung im Waring®-Blender - Eigenschaften der Polymerpartikel

| Bsp. | CRC g/g | AUNL g/g | AUL g/g | AUHL g/g | Feuchtegehalt Gew.-% | Extrahierbare Gew.-% | Vortex s | FSR g/g s | | CRC+AUL g/g | CRC+AUHL g/g |
|---|---|---|---|---|---|---|---|---|---|---|---|
| 1 | 55,0 | 44,3 | 7,2 | 6,6 | 2,2 | 34 | 200 | 0,06 | | 62,2 | 61,6 |
| 1-1 | 46,0 | 59,0 | 26,0 | 9,3 | 1,2 | 5 | 194 | 0,08 | | 72,0 | 55,3 |
| 1-2 | 43,8 | 55,5 | 34,0 | 19,7 | 1,1 | 13 | 192 | 0,12 | | 77,8 | 63,5 |
| 2 | 46,1 | 54,1 | 7,32 | 6,7 | 4,5 | 21 | 96 | 0,27 | | 53,4 | 52,79 |
| 2-1 | 37,5 | 63,8 | 32,9 | 10,7 | 0,6 | 6 | 100 | 0,19 | | 70,4 | 48,2 |

(fortgesetzt)

| Bsp. | CRC g/g | AUNL g/g | AUL g/g | AUHL g/g | Feuchtegehalt Gew.-% | Extrahierbare Gew.-% | Vortex s | FSR g/g s | | CRC+AUL g/g | CRC+AUHL g/g |
|---|---|---|---|---|---|---|---|---|---|---|---|
| 2-2 | 37,5 | 60,4 | 38,7 | 24,3 | 0,8 | 4 | 95 | 0,15 | | 76,2 | 61,8 |
| 3 | 42,8 | 52,0 | 7,4 | 6,5 | 4,4 | 21 | 70 | 0,28 | | 50,2 | 49,3 |
| 3-1 | 38,8 | 61,1 | 36,4 | 17,8 | 1,2 | 17 | 85 | 0,12 | | 75,2 | 56,6 |
| 3-2 | 34,0 | 61,9 | 32,9 | 18,1 | 1,1 | 8 | 91 | 0,15 | | 66,9 | 52,1 |
| 3-3 | 30,4 | 50,0 | 22,5 | 15,1 | 0,8 | 18 | 50 | 0,34 | | 52,9 | 45,5 |
| 3-4 | 29,1 | 42,6 | 22,4 | 14,9 | 1,2 | 21 | 75 | 0,30 | | 51,5 | 43,9 |
| 3-5*) | 41,9 | 52,6 | 7,6 | 7,1 | 2,9 | 22 | 71 | 0,26 | | 49,5 | 49,0 |
| 3-6*) | 29,0 | 49,2 | 26,2 | 14,2 | 1,3 | 21 | 76 | 0,33 | | 55,2 | 43,2 |
| 4 | 48,4 | 54,1 | 7,8 | 7,2 | 4,0 | 20 | 130 | 0,13 | | 56,2 | 55,6 |
| 4-1 | 45,0 | 62,3 | 37,5 | 14,4 | 1,7 | 3 | 120 | 0,07 | | 82,5 | 59,4 |
| 4-2 | 42,8 | 60,2 | 39,3 | 26,2 | 1,2 | 3 | 127 | 0,10 | | 82,1 | 69,0 |
| 5 | 40,7 | 39,3 | 7,5 | 6,6 | 3,5 | 26 | 60 | 0,09 | | 48,2 | 47,3 |
| 5-1 | 40,1 | 53,7 | 18,8 | 7,2 | 2,4 | 16,1 | 50 | 0,14 | | 58,9 | 47,3 |
| 5-2 | 37,8 | 55,3 | 33,0 | 16,3 | 0,9 | 11,2 | 49 | 0,18 | | 70,8 | 54,1 |
| 6 | 64,0 | 9,7 | 6,9 | 6,1 | 2 | 29 | 92 | 0,18 | | 70,9 | 70,1 |
| 6-1 | 45,2 | 53,0 | 26,9 | 12,1 | 1,1 | 18,3 | 90 | 0,13 | | 72,1 | 57,3 |
| 6-2 | 40,5 | 56,6 | 30,3 | 16,3 | 0,9 | 12,0 | 90 | 0,11 | | 70,8 | 56,8 |
| 7*) | 44,3 | 63,9 | 12,1 | 7,1 | 4,0 | 22 | 62 | 0,33 | | 56,4 | 51,4 |
| *) Vergleichsbeispiel | | | | | | | | | | | |

**Patentansprüche**

1.  Verfahren zur Herstellung wasserabsorbierender Polymerpartikel durch Polymerisation einer Monomerlösung, enthaltend

    a) mindestens ein ethylenisch ungesättigtes, säuregruppentragendes Monomer, das zumindest teilweise neutralisiert sein kann,
    b) optional einen oder mehrere Vernetzer,
    c) mindestens einen Initiator,
    d) optional ein oder mehrere mit den unter a) genannten Monomeren copolymerisierbare ethylenisch ungesättigte Monomere und
    e) optional ein oder mehrere wasserlösliche Polymere,

    wobei die Monomerlösung während der Polymerisation in einem hydrophoben organischem Lösungsmittel suspendiert ist, das hydrophobe organische Lösungsmittel bei 23°C eine Löslichkeit in Wasser von weniger als 5 g/100 g aufweist, während oder nach der Polymerisation im hydrophoben organischen Lösungsmittel agglomeriert wird und thermischer Oberflächennachvernetzung der erhaltenen agglomerierten Polymerpartikel mittels eines organischen Oberflächennachvernetzers, **dadurch gekennzeichnet, dass** die Menge an Vernetzer b) so gewählt wird, dass die agglomerierten Polymerpartikel vor der Oberflächennachvernetzung eine Zentrifugenretentionskapazität von mindestens 37 g/g aufweisen und die thermische Oberflächennachvernetzung bei 100 bis 190°C durchgeführt wird.

2.  Verfahren gemäß Anspruch 1, **dadurch gekennzeichnet, dass** nach der Polymerisation im hydrophoben organischen Lösungsmittel agglomeriert wird.

3.  Verfahren gemäß Anspruch 1 oder 2, **dadurch gekennzeichnet, dass** die Polymerisation in Gegenwart eines Kettenübertragungsreagenz durchgeführt wird.

4. Verfahren gemäß einem der Ansprüche 1 bis 3, **dadurch gekennzeichnet, dass** die Menge an Vernetzer b) so gewählt wird, dass die Polymerpartikel vor der Oberflächennachvernetzung eine Zentrifugenretentionskapazität von mindestens 40 g/g aufweisen.

5. Verfahren gemäß einem der Ansprüche 1 bis 5, **dadurch gekennzeichnet, dass** die thermische Oberflächennachvernetzung bei 120 bis 170°C durchgeführt wird.

6. Verfahren gemäß einem der Ansprüche 1 bis 5, **dadurch gekennzeichnet, dass** der organische Oberflächennachvernetzer ausgewählt ist aus Alkylenkarbonaten, 2-Oxazolidinonen, Bis- und Poly-2-oxazolidinonen, 2-Oxotetrahydro-1,3-oxazinen, N-Acyl-2-Oxazolidinonen, zyklische Harnstoffen, bizyklische Amidoacetalen, Oxetanen und Morpholin-2,3-dionen.

7. Verfahren gemäß einem der Ansprüche 1 bis 6, **dadurch gekennzeichnet, dass** bezogen auf die erhaltenen Polymerpartikel von 1 bis 5 Gew.-% an organischem Oberflächennachvernetzer eingesetzt wird.

8. Verfahren gemäß einem der Ansprüche 1 bis 7, **dadurch gekennzeichnet, dass** bei der Polymerisation mindestens ein Dispergierhilfsmittel verwendet wird.

9. Verfahren gemäß einem der Ansprüche 1 bis 8, **dadurch gekennzeichnet, dass** die erhaltenen Polymerpartikel nach der Polymerisation zumindest teilweise azeotrop entwässert werden.

10. Verfahren gemäß Anspruch 9, **dadurch gekennzeichnet, dass** die erhaltenen Polymerpartikel nach der azeotropen Entwässerung filtriert und getrocknet werden.

11. Verfahren gemäß einem der Ansprüche 1 bis 10, **dadurch gekennzeichnet, dass** die thermische Oberflächennachvernetzung in einem Mischer mit bewegten Mischwerkzeugen durchgeführt wird.

12. Wasserabsorbierende Polymerpartikel, erhältlich nach einem Verfahren der Ansprüche 1 bis 11, wobei die wasserabsorbierenden Polymerpartikel eine Zentrifugenretentionskapazität von mindestens 37 g/g, eine Absorption unter einem Druck von 21,0 g/cm$^2$ von mindestens 30 g/g, eine Absorption unter einem Druck von 49,2 g/cm$^2$ von mindestens 14 g/g, eine Summe aus Zentrifugenretentionskapazität und Absorption unter einem Druck von 21,0 g/cm$^2$ von mindestens 69 g/g, eine Summe aus Zentrifugenretentionskapazität und Absorption unter einem Druck von 49,2 g/cm$^2$ von mindestens 54 g/g und weniger als 20 Gew.-% Extrahierbare aufweisen.

13. Wasserabsorbierende Polymerpartikel gemäß Anspruch 12, wobei die wasserabsorbierenden Polymerpartikel eine Zentrifugenretentionskapazität von mindestens 41 g/g aufweisen.

14. Wasserabsorbierende Polymerpartikel gemäß Anspruch 12 oder 13, wobei die wasserabsorbierenden Polymerpartikel eine Absorption unter einem Druck von 21,0 g/cm$^2$ von mindestens 34 g/g aufweisen.

15. Wasserabsorbierende Polymerpartikel gemäß einem der Ansprüche 12 bis 14, wobei die wasserabsorbierenden Polymerpartikel eine Absorption unter einem Druck von 49,2 g/cm$^2$ von mindestens 20 g/g aufweisen.

16. Wasserabsorbierende Polymerpartikel gemäß einem der Ansprüche 12 bis 15, wobei die wasserabsorbierenden Polymerpartikel eine Summe aus Zentrifugenretentionskapazität und Absorption unter einem Druck von 21,0 g/cm$^2$ von mindestens 74 g/g aufweisen.

17. Wasserabsorbierende Polymerpartikel gemäß einem der Ansprüche 12 bis 16, wobei die wasserabsorbierenden Polymerpartikel eine Summe aus Zentrifugenretentionskapazität und Absorption unter einem Druck von 49,2 g/cm$^2$ von mindestens 59 g/g aufweisen.

18. Wasserabsorbierende Polymerpartikel gemäß einem der Ansprüche 12 bis 17, wobei die wasserabsorbierenden Polymerpartikel weniger als 14 Gew.-% Extrahierbare aufweisen.

19. Wasserabsorbierende Polymerpartikel gemäß einem der Ansprüche 12 bis 18, wobei die wasserabsorbierenden Polymerpartikel ein Schüttgewicht von weniger als 1,0 g/cm$^3$ aufweisen.

**20.** Wasserabsorbierende Polymerpartikel gemäß einem der Ansprüche 12 bis 19, wobei der Anteil an Partikeln mit einer Partikelgröße von 300 bis 600 $\mu$m mindestens 30 Gew.-%, beträgt.

**21.** Hygieneartikel, umfassend

(A) eine obere flüssigkeitsdurchlässige Schicht,
(B) eine untere flüssigkeitsundurchlässige Schicht,
(C) eine flüssigkeitsabsorbierende Speicherschicht zwischen der Schicht (A) und der Schicht (B), enthaltend von 0 bis 30 Gew.-% eines Fasermaterial und von 70 bis 100 Gew.-% wasserabsorbierende Polymerpartikel gemäß einem der Ansprüche 12 bis 20,
(D) optional eine Aufnahme- und Verteilschicht zwischen der Schicht (A) und der Schicht (C), enthaltend von 80 bis 100 Gew.-% eines Fasermaterial und von 0 bis 20 Gew.-% wasserabsorbierende Polymerpartikel gemäß einem der Ansprüche 12 bis 20,
(E) optional eine Gewebeschicht direkt über und/oder unter der Schicht (C) und
(F) weitere optionale Komponenten.

## Claims

**1.** A process for producing water-absorbing polymer particles by polymerizing a monomer solution comprising

a) at least one ethylenically unsaturated monomer which bears acid groups and may have been at least partly neutralized,
b) optionally one or more crosslinkers,
c) at least one initiator,
d) optionally one or more ethylenically unsaturated monomers copolymerizable with the monomers mentioned under a) and
e) optionally one or more water-soluble polymers,

the monomer solution suspended in a hydrophobic organic solvent having a solubility in water at 23°C of less than 5 g/100 g during the polymerization being agglomerated during or after the polymerization in the hydrophobic organic solvent, and thermally surface postcrosslinking the resultant agglomerated polymer particles by means of an organic surface postcrosslinker, wherein the amount of crosslinker b) is selected such that the agglomerated polymer particles before the surface postcrosslinking have a centrifuge retention capacity of at least 37 g/g and the thermal surface postcrosslinking is conducted at 100 to 190°C.

**2.** The process according to claim 1, wherein agglomeration is effected in the hydrophobic organic solvent after the polymerization.

**3.** The process according to claim 1 or 2, wherein the polymerization is conducted in the presence of a chain transfer reagent.

**4.** The process according to any of claims 1 to 3, wherein the amount of crosslinker b) is selected such that the polymer particles before the surface postcrosslinking have a centrifuge retention capacity of at least 40 g/g.

**5.** The process according to any of claims 1 to 5, wherein the thermal surface postcrosslinking is conducted at 120 to 170°C.

**6.** The process according to any of claims 1 to 5, wherein the organic surface postcrosslinker is selected from alkylene carbonates, 2-oxazolidinones, bis- and poly-2-oxazolidinones, 2-oxotetrahydro-1,3-oxazines, N-acyl-2-oxazolidinones, cyclic ureas, bicyclic amido acetals, oxetanes and morpholine-2,3-diones.

**7.** The process according to any of claims 1 to 6, wherein from 1 to 5% by weight of organic surface postcrosslinker is used, based on the resultant polymer particles.

**8.** The process according to any of claims 1 to 7, wherein at least one dispersing aid is used in the polymerization.

**9.** The process according to any of claims 1 to 8, wherein the resultant polymer particles are at least partly dewatered azeotropically after the polymerization.

10. The process according to claim 9, wherein the resultant polymer particles are filtered and dried after the azeotropic dewatering.

11. The process according to any of claims 1 to 10, wherein the thermal surface postcrosslinking is conducted in a mixer with moving mixing tools.

12. A water-absorbing polymer particle obtainable by a process of claims 1 to 11, having a centrifuge retention capacity of at least 37 g/g, an absorption under a pressure of 21.0 g/cm$^2$ of at least 30 g/g, an absorption under a pressure of 49.2 g/cm$^2$ of at least 14 g/g, a sum total of centrifuge retention capacity and absorption under a pressure of 21.0 g/cm$^2$ of at least 69 g/g, a sum total of centrifuge retention capacity and absorption under a pressure of 49.2 g/cm$^2$ of at least 54 g/g, and less than 20% by weight of extractables.

13. The water-absorbing polymer particle according to claim 12, having a centrifuge retention capacity of at least 41 g/g.

14. The water-absorbing polymer particle according to claim 12 or 13, having an absorption under a pressure of 21.0 g/cm$^2$ of at least 34 g/g.

15. The water-absorbing polymer particle according to any of claims 12 to 14, having an absorption under a pressure of 49.2 g/cm$^2$ of at least 20 g/g.

16. The water-absorbing polymer particle according to any of claims 12 to 15, having a sum total of centrifuge retention capacity and absorption under a pressure of 21.0 g/cm$^2$ of at least 74 g/g.

17. The water-absorbing polymer particle according to any of claims 12 to 16, having a sum total of centrifuge retention capacity and absorption under a pressure of 49.2 g/cm$^2$ of at least 59 g/g.

18. The water-absorbing polymer particle according to any of claims 12 to 17, having less than 14% by weight of extractables.

19. The water-absorbing polymer particle according to any of claims 12 to 18, having a bulk density of less than 1.0 g/cm$^3$.

20. The water-absorbing polymer particle according to any of claims 12 to 19, wherein the proportion of particles having a particle size of 300 to 600 μm is at least 30% by weight.

21. A hygiene article comprising

(A) an upper liquid-permeable layer,
(B) a lower liquid-impermeable layer,
(C) a liquid-absorbing storage layer between layer (A) and layer (B), comprising from 0% to 30% by weight of a fibrous material and from 70 to 100% by weight of water-absorbing polymer particles according to any of claims 12 to 20,
(D) optionally an acquisition and distribution layer between layer (A) and layer (C), comprising from 80% to 100% by weight of a fibrous material and from 0 to 20% by weight of water-absorbing polymer particles according to any of claims 12 to 20,
(E) optionally a fabric layer directly above and/or beneath layer (C) and
(F) further optional components.

**Revendications**

1. Procédé de préparation de particules polymères absorbant l'eau par polymérisation d'une solution de monomères contenant

a) au moins un monomère éthyléniquement insaturé, portant des groupes acides, qui peut être au moins partiellement neutralisé,
b) éventuellement un ou plusieurs réticulants,

c) au moins un initiateur,

d) éventuellement un ou plusieurs monomères éthyléniquement insaturés copolymérisables avec les monomères mentionnés en a) et

e) éventuellement un ou plusieurs polymères solubles dans l'eau,

la solution de monomères étant en suspension dans un solvant organique hydrophobe pendant la polymérisation, le solvant organique hydrophobe présentant, à 23 °C, une solubilité dans l'eau inférieure à 5 g/100 g, étant agglomérée pendant ou après la polymérisation dans le solvant organique hydrophobe, et postréticulation thermique en surface des particules polymères agglomérées obtenues au moyen d'un postréticulant organique de surface, **caractérisé en ce que** la quantité de réticulant b) est choisie de telle sorte que les particules polymères agglomérées présentent, avant la postréticulation en surface, une capacité de rétention centrifuge d'au moins 37 g/g et la postréticulation thermique en surface est effectuée à une température de 100 à 190 °C.

2. Procédé selon la revendication 1, **caractérisé en ce que** l'agglomération a lieu après la polymérisation dans le solvant organique hydrophobe.

3. Procédé selon la revendication 1 ou 2, **caractérisé en ce que** la polymérisation est réalisée en présence d'un réactif de transfert de chaîne.

4. Procédé selon l'une des revendications 1 à 3, **caractérisé en ce que** la quantité de réticulant b) est choisie de telle sorte que les particules polymères présentent, avant la postréticulation en surface, une capacité de rétention centrifuge d'au moins 40 g/g.

5. Procédé selon l'une des revendication 1 à 5, **caractérisé en ce que** la postréticulation thermique en surface est réalisée à une température de 120 à 170 °C.

6. Procédé selon l'une des revendications 1 à 5, **caractérisé en ce que** le postréticulant organique de surface est choisi parmi les carbonates d'alkylène, les 2-oxazolidinones, les bis-et poly-2-oxazolidinones, les 2-oxotétrahydro-1,3-oxazines, les N-acyl-2-oxazolidinones, les urées cycliques, les amidoacétals bicycliques, les oxétanes et les morpholine-2,3-diones.

7. Procédé selon l'une des revendications 1 à 6, **caractérisé en ce qu'**on utilise 1 à 5 % en poids, par rapport aux particules polymères obtenues, de postréticulant organique de surface.

8. Procédé selon l'une des revendications 1 à 7, **caractérisé en ce qu'**au moins un adjuvant de dispersion est utilisé lors de la polymérisation.

9. Procédé selon l'une des revendications 1 à 8, **caractérisé en ce que** les particules polymères obtenues sont déshydratées de manière azéotrope, au moins en partie, après la polymérisation.

10. Procédé selon la revendication 9, **caractérisé en ce que** les particules polymères obtenues sont filtrées et séchées après la déshydratation azéotrope.

11. Procédé selon l'une des revendications 1 à 10, **caractérisé en ce que** la postréticulation thermique en surface est réalisée dans un mélangeur pourvu d'outils de mélange en mouvement.

12. Particules polymères absorbant l'eau pouvant être obtenues par un procédé selon les revendications 1 à 11, les particules polymères absorbant l'eau présentant une capacité de rétention centrifuge d'au moins 37 g/g, une absorption sous une pression de 21,0 g/cm$^2$ d'au moins 30 g/g, une absorption sous une pression de 49,2 g/cm$^2$ d'au moins 14 g/g, une somme de la capacité de rétention centrifuge et de l'absorption sous une pression de 21,0 g/cm$^2$ d'au moins 69 g/g, une somme de la capacité de rétention centrifuge et de l'absorption sous une pression de 49,2 g/cm$^2$ d'au moins 54 g/g et moins de 20 % en poids de substances extractibles.

13. Particules polymères absorbant l'eau selon la revendication 12, les particules polymères absorbant l'eau présentant une capacité de rétention centrifuge d'au moins 41 g/g.

14. Particules polymères absorbant l'eau selon la revendication 12 ou 13, les particules polymères absorbant l'eau présentant une absorption sous une pression de 21,0 g/cm$^2$ d'au moins 34 g/g.

**15.** Particules polymères absorbant l'eau selon l'une des revendications 12 à 14, les particules polymères absorbant l'eau présentant une absorption sous une pression de 49,2 g/cm$^2$ d'au moins 20 g/g.

**16.** Particules polymères absorbant l'eau selon l'une des revendications 12 à 15, les particules polymères absorbant l'eau présentant une somme de la capacité de rétention centrifuge et de l'absorption sous une pression de 21,0 g/cm$^2$ d'au moins 74 g/g.

**17.** Particules polymères absorbant l'eau selon l'une des revendications 12 à 16, les particules polymères absorbant l'eau présentant une somme de la capacité de rétention centrifuge et de l'absorption sous une pression de 49,2 g/cm$^2$ d'au moins 59 g/g.

**18.** Particules polymères absorbant l'eau selon l'une des revendications 12 à 17, les particules polymères absorbant l'eau contenant moins de 14 % en poids de substances extractibles.

**19.** Particules polymères absorbant l'eau selon l'une des revendications 12 à 18, les particules polymères absorbant l'eau présentant une masse volumique apparente inférieure à 1,0 g/cm$^3$.

**20.** Particules polymères absorbant l'eau selon l'une des revendications 12 à 19, la proportion de particules présentant une taille de particule de 300 à 600 $\mu$m représentant au moins 30 % en poids.

**21.** Article d'hygiène, comprenant

(A) une couche supérieure perméable aux liquides ;
(B) une couche inférieure imperméable aux liquides ;
(C) une couche d'accumulation absorbant les liquides entre la couche (A) et la couche (B), contenant 0 à 30 % en poids d'un matériau fibreux et 70 à 100 % en poids de particules polymères absorbant l'eau selon l'une des revendications 12 à 20,
(D) éventuellement une couche de réception et de répartition entre la couche (A) et la couche (C), contenant 80 à 100 % en poids d'un matériau fibreux et 0 à 20 % en poids de particules polymères absorbant l'eau selon l'une des revendications 12 à 20,
(E) éventuellement une couche de tissu directement au-dessus et/ou au-dessous de la couche (C) et

F) d'autres composants éventuels.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP S63218702 A
- WO 2006014031 A1
- WO 2008068208 A1
- US 20110238026 A1
- US 20110059329 A1
- WO 2015110321 A1
- WO 2015062883 A2
- WO 2008026783 A1
- EP 1433526 A2
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2
- WO 9924525 A1
- EP 1321182 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- DE 3713601 A1
- US 8003210 B

**In der Beschreibung aufgeführte Nicht-Patentliteratur**

- **F.L. BUCHHOLZ** ; **A.T. GRAHAM**. Modern Superabsorbent Polymer Technology. Wiley-VCH, 1998, 69-117